# EUROPEAN PATENT SPECIFICATION

(11) **EP 2 051 703 B1**
(45) Date of publication and mention of the grant of the patent: **21.07.2010**
(21) Application number: 06820527.7
(22) Date of filing: 14.12.2006
(51) Int. Cl.: A61K 9/24, A61K 31/496, A61K 31/536, A61K 31/5513, A61K 31/7068, A61K 31/7072, A61K 31/7076, A61K 45/06, A61P 31/18

(54) **PHARMACEUTICAL COMBINATION COMPRISING NUCLEOTIDE AND NUCLEOSIDE REVERSE TRANSCRIPTASE INHIBITORS (SUCH AS TENOFOVIR AND LAMIVUDINE) IN DIFFERENT PARTS OF THE DOSAGE UNIT**
PHARMAZEUTISCHE KOMBINATION AUS NUCLEOTID UND NUCLEOSID-REVERSE-TRANSKRIPTASE-HEMMERN (WIE TENOFOVIR UND LAMIVUDIN) IN VERSCHIEDENEN TEILEN DER DOSIEREINHEIT
COMBINAISON PHARMACEUTIQUE CONTENANT DES INHIBITEURS DE LA TRANSCRIPTASE INVERSE NUCLEOSIDIQUE ET NUCLEOTIDIQUE (COMME LE TENOFOVIR ET LA LAMIVUDINE) DANS DIFFERENTES PARTIES DE L'UNITE DE DOSAGE

(30) Priority: 14.12.2005 IN MU15662005; 13.11.2006 IN MU18782006
(43) Date of publication of application: 29.04.2009
(73) Proprietor: CIPLA LIMITED, Bombay 400 008 (IN)
(72) Inventor: LULLA, Amar, Maharashtra (IN); MALHOTRA, Geena, Maharashtra (IN)
(74) Representative: Cottrill, Emily Elizabeth Helen
(86) International application number: PCT/GB2006/004687
(87) International publication number: WO 2007/068934

(56) References cited:
- WO-A-00/16755
- WO-A-2005/021001
- WO-A-2006/135933
- WO-A-2007/013047
- GILEAD: "Gilead Provides Update on Development of Fixed-Dose Regimen of Truvada (emtricitabine and tenofovir disoproxil fumarate) and Sustiva (efavirenz)" INTERNET CITATION, [Online] 26 April 2005 (2005-04-26), XP002417804 Retrieved from the Internet: URL:http://investors.gilead.com/phoenix.zh tml?c=69964&p=irol-newsArticle& t=Regular&id=701414&> [retrieved on 2007-01-31]
- DATABASE WPI Week 200357 Derwent Publications Ltd., London, GB; AN 2003-608501 XP002461780 & ZA 200 110 501 A (CIPLA MEDPRO PTY LTD) 29 May 2002 (2002-05-29)
- GILEAD ET AL: "Atripla" INTERNET CITATION, [Online] July 2006 (2006-07), XP002417853 Retrieved from the Internet: URL:http://www.fda.gov/cder/foi/label/2006 /021937lbl.pdf> [retrieved on 2007-01-31]
- FDA: "Guidance for Industry Fixed Dose Combination and Co-Packaged Drug Products for Treatment of HIV" INTERNET CITATION, [Online] May 2004 (2004-05), XP002417855 Retrieved from the Internet: URL:http://www.fda.gov/oc/initiatives/hiv/ hivguidance.html> [retrieved on 2007-01-31]

## Description

The present invention relates to a pharmaceutical formulation for the treatment of human immunodeficiency virus (HIV) infection. In particular, the invention relates to a pharmaceutical formulation comprising a nucleoside reverse transcriptase inhibitor and a nucleotide reverse transcriptase inhibitor and to a pharmaceutical product containing the pharmaceutical formulation and a non-nucleoside reverse transcritase inhibitor. There is also described a process for preparing the pharmaceutical formulation and to its use in therapy.

Human Immunodeficiency virus (HIV) infection and related diseases are a major and global problem in today's world. HIV is the etiological agent of the complex disease that includes progressive suppression or destruction of the immune system known as Acquired Immune Deficiency Syndrome or AIDS and degeneration of the cental and peripheral nervous system. HIV also predisposes subjects to fatal opportunistic inactions.

HIV's genetic material is stored in the form of RNA To allow incorporation of this genetic material into the host cell DNA, this viral PNA needs to be transformed into viral DNA. HIV is known as a retrovirus because it has this capability of copying RNA into DNA. Reverse transcriptase is a necessary enzyme for this reaction. To build its viral DNA, HIV uses nucleotides from the host cell's cytoplasm.

Nucleoside reverse transcriptase inhibitors (NRTIs) are nucleoside analogues that lack a 3' hydroxyl group. After these nucleoside, analogues have been phosporylated (to the corresponding nucleotide), they can be incorporated into the growing DNA chain. Because of the missing 3' hydroxyl group in these analogues, the newly made DNA strand is terminated early and polymerization by the reverse transcriptase is stopped. The activity of these NRTIs is not limited to HIV reverse transcriptase only, but can be used against other retroviruses also.

Tenofovir is a new nucleotide reverse transcriptase inhibitor recently approved in the United States for the treatment of HIV-1 infection in combination with other antiretroviral agents. Nucleotide analogues are very similar to nucleoside analogues but are prephosphorylated, and thus require less processing by the body. Tenofovir DF (disoproxil fumarate) is described in US patent no. 5,935,946,_5,922,695, 5,977,089, 6,043,230 & 6,069,249 while PMPA or Tenofovir DF is described in US patent nos. 4,808,716, 5,733,788 & 6,057,305.

US2004/0224917 describes the combination of Tenofovir DF and Emtricitabine:

A common feature of retrovirus replication is the extensive post-translation processing of precursor polyproteins by a virally encoded protease to generate mature Viral proteins required for virus assembly and function. Inhibition of this processing prevents the production of normally infectious virus. Literature reports that genetic inactivation of the HIV encoded protease results in the production of immature, non-infectious virus particles. These results indicate that inhibition of the HIV protease represents a viable method for the treatment of AIDS and the prevention or treatment of infection by HIV.

One substantial and persistent problem in the treatment of AIDS has been the ability of the HIV virus to develop resistance to the individual therapeutic agents employed to treat the disease.

Nowadays various combinations have been made available for this purpose and several attempts have been made to formulate combination regimens. One example is the combination of synthetic nucleoside analogues Lamivudine (150mg) and Zidovudine (300mg), which is commercially available as Combivir® of GlaxoSmithKline. Another such combination is of the nucleoside analogues Abacavir and Lamivudine, which is described in Glaxo's patent application no WO03/101467.

Lamivudine (also known as 3TC) and its use in the treatment and prophylaxis of viral infections are described in US 5,047,407. Lamivudine and its use against HIV are described in WO 91/17159 and EP 0382526. Crystalline forms of lamivudine are described in WO 92/21676.

Combinations of lamivudine with other nucleoside reverse transcriptase inhibitors, in particular zidovudine AZT, are described in WO 92/20344, WO 98/18477, and WO/9955372.

Various non-nucleoside reverse transcriptase inhibitors (NNRTIs), are known, such as Delavirdine, Capravirine, Efavirenz and Nevirapine. NNRTIS are common components of therapy for antirefroviralnaive HIV-infected patients, and provide synergistic activity with nucleoside reverse transcriptase inhibitors (NRTIs).

Efavirenz is chemically known as (S)-6-chloro-4-(cycopropylethynyl)-1, 4-dihydro-4-(trifluoromethyl)-2H-3, 1-benzoxazin-2-one. Efavirenz is a HIV-1 specific, non nucleoside, reverse transcriptase inhibitor. Efavirenz is useful for the treatment of HIV and has been reported to inhibit reproduction of HIV in the body.

Efavirenz is commercially available from Bristol-Myers Squibb Co, under the name SUSTIVA®, for treatment of HIV, and is described, for example, in US patents 5,519,021, 5,663,1699, 5,811,423 and 6,238, 695.

Nevirapine, chemically, 11-Cyclopropyl-5,11-dihydro-4-methyl-6H-dipyrido[3,2-b: 2', 3'-e][1,4]diazepin-6-one is a non-nucleoside reverse transcriptase inhibitor. The therapeutic uses of nevirapine and related compounds and their preparations are described in U. S. Patent No. 5,366,972. Nevirapine is commercially available as 200 mg tablet and 50 mg/5 mL in 240 mL oral suspension. It is sold under the name VIRAMUNE®.

Combination therapy reduces the daily dosages to be taken by patients and simplifies dosing schedule thereby increases patient compliance. Combination therapy also_increases the drug efficacy. Use of combination therapy can yield an equivalent antiviral effect with reduced toxicity.

In spite of the existence of such combinations, there still remains a need to develop a combination for acute therapy and for resistant HIV viruses. It is thus an object of the present invention to provide a pharmaceutical composition which, inter alia, assists in treating the human immunodeficiency virus (HIV), and optionally related disorders resulting in AIDS.

The present invention provides an effective combination which solves or alleviates the problems of the prior art. In particular, the present invention provides a pharmaceutical formulation in a single unit dosage form which has increased patient compliance and improved stability.

In a first aspect, the invention provides a pharmaceutical formulation in a single unit dosage form, wherein the dosage form comprises:
(a) a nucleoside reverse transcriptase inhibitor, and
(b) a nucleotide reverse transcriptase inhibitor,
wherein the nucleoside reverse transcriptase inhibitor is provided in a different region of the dosage form to the nucleotide reverse transcriptase inhibitor.

In a second aspect, there is provided a pharmaceutical product comprising a pharmaceutical formulation according to the invention, and further comprising a non-nucleoside reverse transcriptase inhibitor.

There is described a pharmaceutical product comprising:
i) lamivudine,
ii) a nucleotide reverse transcriptase inhibitor, and
iii) a non-nucleoside reverse transcriptase inhibitor.

In another aspect there is provided the use of a nucleoside reverse transcriptase inhibitor or physiologically functional derivative thereof and a nucleotide reverse transcriptase inhibitor in the manufacture of a unitary dosage form pharmaceutical for the treatment or prevention of symptoms or effects of an HIV infection in an infected individual, wherein the nucleoside reverse transcriptase inhibitor is provided in a different region of the dosage form to the nucleotide reverse transcriptase inhibitor.

There is described the use of a pharmaceutical formulation according to the invention, in the manufacture of a medicament for the treatment or prevention of symptoms or effects of an HIV infection in an infected individual.

The present invention further provides pharmaceutical compositions for simultaneous, separate or sequential use in the treatment or prevention of viral infections. In particular, the invention providers a pharmaceutical product comprising a pharmaceutical formulation according to invention and a non-nucleoside reverse transcriptase inhibitor for simultaneous, separate on sequential use.

The present invention thus allows an efficacious and long lasting therapy for AIDS which lowers HIV levels in patients to undetectable level and raises CD4 cell counts for prolonged periods without the development of resistance.

The combination therapies are a step shead in the art for enhancing the effectiveness in treating AIDS and to preclude the development of resistance to individual therapeutic agents.

In the first aspect of the invention, the nucleoside reverse transcriptase inhibitor is preferably chosen from lamivudine, abacavir, emtricitabine, zidovudine or stavudine. Preferably lamivudine is used. The nucleotide reverse transcriptase inhibitor is preferably chosen from tenofovir DF or adefovir, and is preferably tenofovir DF.

In the second aspect of the invention, the non-nucleoaide reverse transcriptase inhibitor is preferably chosen from efavirenz, nevirapine or delavirdine. Preferably the non-nucleoside reverse transcriptase inhibitor is efavirenz.

In the third aspect of the invention, the nucleotide reverse transcriptase inhibitor is preferably chosen from tenofovir DF or adefovir. Preferably tenofovir DR is used. The non-nucleoside reverse transcriptase inhibitor is preferably chosen from efavirenz, nevirapine, delavirdine. Preferably the non-nucleoride reverse transcription inhibitor is ofavirenz.

The composition may be provided as an oral dosage form.

The composition or product according to the invention may be useful in the treatment of viral infections, particularly retroviral infections, which may include human immunodeficiency virus (HIV) and related disorders resulting in AIDS.

A layered tablet (bilayered, trilayered, etc.) as used herein refers to a tablet in which two or more layers of active material have been compressed successively. Such tablets are also known as laminated tablets. Both or all layers of active material are exposed (although the tablet may be further coated). This differs from a core or coated core tablet, in which the core of a first active material is circumscribed, and thus concealed from the exterior of the tablet, by a coating layer of another active material.

Further, there is described a method for treating, reducing or inhibiting retroviral infections, in particular HIV infections in a mammal, which includes administering to a human, a safe and effective amount of a pharmaceutical formulation or product according to the invention.

Reference to word "treatment" as used herein extends to both the prophylaxis and the treatment of an established malady, infection or its symptoms.

HIV causes a variety of clinical conditions including acquired immunodeficiency syndrome (AIDS) and chronic neurological disorders. Multiple drug regimes dramatically improve the treatment of HIV infected patients.

Single drug treatment regimens typically require long term treatment increasing the evidence of unwanted side effects. Moreover, single drug therapies are particularly vulnerable to mutation in the HIV runs, leading to drug resistant variants of HIV.

The use of multiple drug therapies may reduce the development of drug resistant strains of HIV because one drug will usually cancel out mutations against other drug. Multiple drug therapies even inhibit replication of HIV viruses for a period of time sufficient to eliminate HIV from the body. However, not all combinations of drugs are equally efficacious, and some combinations can be ineffective or have undesirable side effects. Moreover, certain drug combinations can result in undesirably poor stability.

The effective multiple drug treatments for HIV often require strict compliance with a complex treatment regimen that can require the administration of many different drugs per day, administered at a precisely timed intervals with careful attention to diet. Patient non-compliance is a well-known problem accompanying such complex treatment regimens in the treatment of HIV because such non-compliance may lead to the emergence of multiple drug resistant strains of HIV and also abandonment of treatment in the middle of the therapy.

The combination therapy thus provides a method to enhance the effectiveness in treating AIDS and to prevent the development of resistance to the individual therapeutic agents.

It will be appreciated, therefore, the the pharmaceutical combinations of the present invention, and in particular the preferred combination of lamivadine, tenofovir DF, and efavirenz, provide significant advantages over the prior art. The combination may conveniently be presented as individual pharmaceutical formulations in unitary dosage form. In this respect the present invention provides a pharmaceutical kit or product comprising (i) a first pharmaceutical formulation comprising lamivudine, together with one or more pharmaceutically acceptable carriers or excipients, and tenofovir DF together with one or more pharmaceutically acceptable carriers or excipients; and (ii) a second pharmaceutical formulation comprising efavirenz or a physiologically acceptable salt thereof, together with one or more pharmaceutically acceptable carriers or excipients; for separate or sequential use in the treatment or prevention of viral infections.

The pharmaceutical kit or product may be provided in a patient pack, optionally comprising an information insert containing directions on the use of the kit/product.

Lamivudine (also known as 3TC) is a synthetic nucleoside analogue, chemically known as (2R,cis)-4-amino-1-(2-hydroxymethyl-1,3-oxathiolan-5-yl)-(1H)-pyrimidin-2-one (Epivir®). Lamivudine has proven antiviral activity against HIV and other viruses such sa HBV.

Intracellularly, lamivudine is phosphorylated to its active 5'-triphosphate metabolite, lamivudine triphosphate (L-TP). The principal mode of action of L-TP is the inhibition of HIV-1 reverse transcriptase (RT) via DNA chain termination after incorporation of the nucleoside analogue into viral DNA. L-TP is a weak inhibitor of mammalian DNA polymerases (alpha) and (beta), and mitochondrial DNA polymerase (gamma).

Lamivudine has also been referred to as (-)-1-[(2R, 5S) 2-(Hydroxymethyl)-1,3-oxathiolan-5-yl] cystosine, (Hydroxymethyl)-1,3-oxathiolan-5-yl] cystosine and it has proven antiviral activity.against human immunodeficiency virus (HIV) and other viruses such as hepatitis B. Lamivudine is commercially available from *Glaxo Wellcome Inc* under trade name EPIVIR.

Tenofovir disoproxil fumarate is also known as PMPA. Tenofovir DF (a prodrug of tenofovir) is a fumaric acid salt of bis-isopropoxycarbonyloxymethyl ester derivative of tenofovir. Tenofovir disoproxil fumarate is 9-[(R)-2-[[bis[[(isopropoxycarbonyl) oxy] methoxy] phosphinyl] methoxy] propyl] adenine fumarate (1:1). Tenofovir disoproxil fumarate requires initial diester hydrolysis for conversion to tenofovir and subsequent phosphorylations by cellular enzymes to form tenofovir diphosphate. Tenofovir diphosphate inhibits the activity of HIV reverse transcriptase by competing with the natural substrate deoxyadenosine 5'-triphosphate and, after incorporation into DNA, by DNA chain termination. Tenofovir diphosphate is a weak inhibitor of mammalian DNA polymerases alpha & beta and of mitochondrial DNA polymerase.

Tenofovir disoproxil fumarate is an analog of adefovir and is classified as a nucleotide reverse transcriptase inhibitor (NtRTI). Tenofovir DF is a competitive inhibitor of other naturally occurring nucleotides, and its ultimate biological activity is viral DNA chain termination. Tenofovir DF is a novel nucleotide analog with antiviral activity against both HIV and HBV. The mechanism of tenofovir DF is similar to that of nucleoside analogs, which interferes with reverse transcriptase and prevents translation of viral genetic material into viral DNA. Unlike the nucleoside analogs, the nucleotide reverse transcriptase inhibitors are chemically pre-activated with the presence of phosphate group. Since the phosphorylation step is not necessary, nucleotide analogs can incorporate into viral DNA chain more rapidly than nucleoside analogs. More importantly, this will bypass a viral mechanism of nucleoside resistance. Tenofovir DF is commercially available from Gilead Science Inc. under the trade name VIREAD®.

The chemical stability of the active ingredients in a pharmaceutical formulation is of significant concern, as is the appearance of the formulation and how it changes over time. The inventors of the present invention have surprisingly found that Lamivudine and Tenofovir DF, when intimately mixed to form a (single layered) tablet showed undesirable properties in stability testing. The appearance of tablets changed to brown colour at Controlled Room Temperature (25°) and even at Accelerated temperature (40°C). However, the inventors have surprisingly found that such a change in appearance is not found in case of a bilayered tablet.

| Product: Lamivudine & Tenofovir Disoproxil Fumarate Tablets | | | |
|---|---|---|---|
| CONDITION PARAMETERS | INITIAL | 25°C/60% RH | 40°C75%RH |
| | | 1M | 1M |
| Appearance | Single layered tablets | Brown colouration | Brown colouration with gas formation. |
| Appearance | Bilayered tablets | No change in the colour | No change in the colour |

By means of a pharmaceutical formulation according to the present invention comprising a bilayered tablet comprising an NRTI and an NtRTI, treatment regimens for HIV and other viruses can be simplified with the goal of enhancing patient compliance by providing a simplified dosage therapy.

It is also possible to combine any two of the NRTIs and NtRTIs employed in the present invention in a unitary dosage form for separate or sequential administration with a separate dosage form comprising an NNRTI. In a preferred embodiment, a typical unitary dosage may contain lamivudine and tenofovir DF, and a further unitary dosage may contain efavirenz. In this respect, the present invention provides a pharmaceutical product comprising (i) a first pharmaceutical formulation comprising lamivudine and tenofovir DF, in the form of a bilayered tablet, together with one or more pharmaceutically acceptable carriers or excipients; and (ii) a second pharmaceutical formulation comprising efavirenz, together with one or more pharmaceutically acceptable carriers or excipients, for separate or sequential use in the treatment or prevention of viral infections, in particular retroviral infections, and especially the symptoms or effects of HIV infection, in an infected animal.

The pharmaceutical product and formulation of the present invention employ a combination of safe and therapeutically effective amount of at.least two therapeutically active agents and preferably three therapeutically active agents, such as a safe and therapeutically effective amount of 2',3'-dideoxy-3'-thiacytidine (lamivudine), a safe and therapeutically effective amount of tenofovir DF, (R)-9-(2-phosphonylmethoxypropyl) adenine, optionally with a safe and therapeutically effective amount of efavirenz, along with safe and effective amounts of pharmaceutically acceptable excipients to maintain the homogeneity of the dosage forms.

The composition may be provided in the form of tablets or capsules.

The pharmaceutical product of the present invention conveniently allows administration of a pharmaceutical combination a separate or subsequent dosage containing three active compounds in oral dosage forms containing specific dosage ranges for each compound. Preferably a unity dosage form is provided comprising an NRTI and an NtRTI. Preferably an additional dosage form is provided comprising an NNRTI, for separate or sequential administration.

Lamivudine may be present preferably in a range of 5-600 mg and most preferably 300 mg per unit dosage form.

Tenofovir DF may be present preferably in a range of 75-600 mg and preferably 300 mg per unit dosage form.

Efavirenz may be present preferably in a range of 50 - 600 mg and preferably 600 mg per unit dosage form.

The formulation and product of the present invention may further comprise pharmaceutical excipients to impact beneificial characteristics to the dosage form. Typical excipients include, diluents or bulking agents, fillers, disintegrants, binders, lubricants, coating materials, wetting agents and the like.

Where present, a diluent or bulking agent can be selected to provide an increase in tablet size. The skilled person can utilize known methods to select a bulking agent, which provides hardness friability and disintegration time required for pharmaceutical advantage. Suitable diluents included microcrystalline cellulose, lactose and the like. The diluent is preferably present in an amount of from 5% to 50% by weight of the formulation.

Fillers suitable for use with the present invention may comprise one or more of sugars, sugar alcohols, starches, and inert materials, such as kaolin and the like, that add to the bulk of the formulation.

Disintegrating agents suitable for use with the present invention may comprise one or more of celluloses and their derivatives, alginates, agar-agar, certain complex silicilates, starches, modified starches and their derivatives, polyvinylpyrolidones and the like. Preferred disintegrants include sodium starch glycollate and/or croscarmellose sodium. The disintegrant is preferably present in an amount of from 0.5% to 30% by weight of the formulation.

Binders may comprise one or more of but not limited to, natural and synthetic gums, celluloses, starches, gelatins and povidones and the like. Preferably, the binder comprises starch, Maltodextrins, HPMC, HPC and/or povidone. The binder is preferably present in an amount of from 1% to 50% by weight of the formulation.

Lubricants suitable for use with the present invention may comprise one or more of talc, magnesium stearate, starch, dextrin, sodium stearyl fumarate, hydrogenated vegetable oils, polyethylene glycols and their derivatives, sodium lauryl sulphate and the like. Preferably, the lubricants comprise one or more of magnesium stearate, zinc stearate, calcium stearate and sodium stearyl fumarate. More preferably the lubricant is magnesium stearate. The lubricant is preferably present in an amount of from 0.25% to 3% by weight of the formulation.

The tablets may be coated for the purpose of providing protection from moisture. The coating material can be selected from one or more of celluloses and their derivatives, polyethylene glycols and their derivatives, fatty acids such as stearic acid and their derivatives, and waxes, among other suitable coating materials well known in the art.

The person skilled in the art may include wetting agent such as polysorbate 80, SLS, sucrose esters, polyethylene glycols, lutrols, cremophor and the like. Where present, the wetting agent is preferably present in an amount of from 0.1% to 5%, by weight of the formulation.

In a preferred embodiment, the formulation is in the form of a bilayered tablet. In another preferred embodiment the product is in the form of a bilayered tablet and a subsequent unitary tablet formulation.

In these embodiments, the first layer of said bilayered tablet preferably contains 5 to 55% wt. lamivudine, 1 to 50 % wt. diluents, 1 to 50 % wt. binders, 1 to 30% wt. disintegrant and 0.25 to 3.0 % wt. of a lubricant.

More preferably, the first layer of tablet containing lamivudine or a physiologically functional derivative thereof in the formulation or product according to the present invention comprises 5 to 55% wt. of lamivudine, 10 to 50 % wt. microcrystalline cellulose, 2 to 30% wt. Sodium starch glycolate, 1 to 10% wt. of starch and 0.25 to 2.5 % wt. of a magnesium stearate.

In these embodiments the second layer of said bilayered tablet preferably contains 10 to 85% wt. of tenofovir DF 1 to 50 % wt. diluent, 1 to 50 % wt. binder, 0.5 to30 % wt. disintegrant and 0.25 to 3 % wt. of a lubricant.

More preferably, the second layer of tablet comprises 35 to 85 % wt. of tenofovir DF, 5 to 50 % wt. lactose or microcrystalline cellulose, 1 to 10 % wt. starch, 1 to 20% wt. sodium starch glycollate and 0.2 to 2 % wt. of magnesium stearate.

In the preferred pharmaceutical product of the present invention, a unitary tablet dose is preferably provided containing at least one non-nucleoside reverse transcriptase inhibitor or a physiologically functional derivative thereof. In the preferred product according to the present invention said unitary dosage form comprises 10 to 50 % wt. of efavirenz 1 to 50 % wt. diluent, 1 to 50 % wt. binder, 0.5 to 30 % wt. disintegrant and 0.2 to 3 % wt. of a lubricant.

Different techniques known in the art may be employed to formulate granules. In a preferred embodiment of the present invention, a method of preparing a pharmaceutical composition for a first layer of said bilayered tablet preferably includes the steps of blending a diluent and disintegrant with lamivudine; granulating it further with water and suitable binder into granules; drying the resulting granules; sizing and lubricating the granules.

A preferred method of preparing a pharmaceutical composition for a second layer of said bi layered tablet preferably includes the steps of blending a diluent with Tenofovir DF; granulating it further with water and suitable binder into granules; drying the resulting granules and sizing; again blending with suitable colour and disintegrant; lubricating the granules.

Lubricated granules of both the layers may then be compressed together using suitable compression machine.

Both the layers may optionally contain colouring agents.

A preferred method of manufacturing the unitary tablet preferably includes the steps of blending a diluent with efavirenz; granulating it further with water and suitable binder into granules; drying the resulting granules and sizing; blending with suitable colour (if desired) and disintegrant; lubricating the granules.

The present invention may be formulated as a multi-layered tablet formulation, preferably bilayered which can typically be administered to patients and permits or achieves delivery of pharmaceutically active agents effective for the prevention or treatment of infection by HIV and in prevention or treatment of the resulting acquired immunodeficiency syndrome (AIDS).

The present invention formulated as a bilayered tablet may be further modified to act as a dispersible tablet comprising suitable excipients known to the person skilled in the art.

Each part of the tablet of the present invention can be prepared by wet granulation or direct compression or dry granulation. In view of the relatively high dosage of lamivudine it is preferred to use wet granulation techniques.

The formulation of the present invention may also be formulated as a trilayered tablets. In this embodiment, a placebo layer is used as an intermediate layer between the NNRT and NtRTI layers. Preferably the placebo layer comprises pharmaceutical excipients but does not comprise any active ingredient. One suitable placebo comprises silica gel. This may further increase the stability of a tablet containing, for example, lamivudine and tenofovir DF in distinct layers.

In a preferred embodiment of the present invention, a pharmaceutical product comprises a bilayered system with a subsequent unitary tablet formulation including the pharmaceutically active agents effective for the treatment of HIV. More particularly, a product according to the present invention preferably comprises bilayered formulation comprising a first layer comprising at least one nucleoside reverse transcriptase inhibitor, optionally further comprising pharmaceutical excipients, and a second layer comprising at least nucleotide reverse transcriptase inhibitor, optionally further comprising pharmaceutical excipients; and a formulation comprising a non-nucleoside reverse transcriptase inhibitor wheren the bilayered formulation form a pharmaceutically stable preparation together.

The invention will now be described further with reference to the following examples.

### Example I

### (A) Lamivudine & Tenofovir DF (disoproxil fumarate) tablet

| INGREDIENTS | QTY (mg /tab) |
|---|---|
| Layer I | |
| Lamivudine | 150.00 |
| Microcrystalline cellulose | 99.50 |
| Sodium starch glycollate | 40.00 |
| Magnesium stearate | 4.00 |
| Color | 5.00 |

| Layer II | |
|---|---|
| Tenofovir DF | 300.00 |
| Lactose | 170.00 |
| Microcrystalline cellulose | 95.00 |
| Starch | 30.00 |
| Polysorbate 80 | 6.50 |
| Croscarmellose sodium | 25.00 |
| Magnesium stearate | 6.50 |
| Colour | 0.50 |

### (B) Efavirenz tablet

| INGREDIENTS | QTY (mg /tab) |
|---|---|
| **Intra-granular ingredients :** | |
| Efavirenz | 600.00 |
| Lactose | 495.00 |
| Sodium starch glycolate | 50.00 |

| **Binder:** | |
|---|---|
| Povidone (PVP K-30) | 40.00 |
| Purified water | q.s. |

| **Extra granular ingredients :** | |
|---|---|
| Sodium starch glycolate | 50.00 |
| Pregelatinised starch | 50.00 |
| Magnesium stearate | 15.00 |
| Colour | 15.00 |
| Purified water | q.s. |

### Process for preparation of bilayer tablet

The preparation of layer - I includes the steps of blending of diluent, disintegrant and optionally suitable colour with Lamivudine and then lubricating the blend.
The preparation of layer-II includes the steps of blending of diluent with Tenofovir DF; further granulating it with water and suitable binder to obtain granules; drying the resulting granules and sizing the granules; again blending the said granules with suitable colour and disintegrant; and lubricating the granules.
Lubricated granules of both the layers then compressed together using suitable compression machine.

Preparation Efavirenz tablet includes the step of blending of diluent with Efavirenz; further granulating it with water and suitable binder to obtain granules; drying the resulting granules and sizing the granules; again blending the said granules with suitable colour and disintegrant; and lubricating the granules.

### Example 2

| INGREDIENTS | QTY (mg /tab) |
|---|---|
| Lamivudine Layer | |
| Lamivudine | 300.00 |
| Microcrystalline cellulose | 98.40 |
| Sodium starch glycollate | 50.00 |
| Colour | 0.60 |
| Starch (binder) | 15.00 |
| Purified water | q.s. |
| Magnesium stearate | 6.00 |

| Tenofovir Layer | |
|---|---|
| Tenofovir disoproxil fumarate equivalent to Tenofovir disoproxil | 300.00 |
| Lactose | 159.50 |
| Croscarmellose sodium | 40.00 |
| Starch | 45.00 |
| Polysorbate 80 | 3.00 |
| Water | q.s. |
| Microcrystalline cellulose | 100.00 |
| Magnesium stearate | 12.50 |

Process of manufacture for Tenofovir layer - A premix of tenofovir and lactose is prepared. This is drymixed with croscarmellose sodium and starch. A binder solution of starch and polysorbate 80 in purified water is prepared. The drymix is granulated using the binder solution, wet granules are dried and sized and lubricated.

For the lamivudine layer - Drymix of lamivudine, microcrystalline cellulose, sodium starch glycollate and colour is prepared. This is granulated with a binder solution (starch paste). The granules are sized, dried and lubricated.

The tablets are compressed and coated using a colour redimix solution.

## Claims

1. A pharmaceutical formulation in a single unit dosage form, wherein the dosage form comprises:
(a) a nucleoside reverse transcriptase inhibitor, and
(b) a nucleotide reverse transcriptase inhibitor,
wherein the formulation is in the form of a multi-layered tablet or a coated core tablet., and wherein the nucleoside reverse transcriptase inhibitor is provided in a different region of the dosage form to the nucleotide reverse transcriptase inhibitor.

2. A pharmaceutical formulation according to claim 1, wherein the nucleoside reverse transcriptase inhibitor is provided in a first region of the dosage form, and the nucleotide reverse transcriptase inhibitor provided in a second region of the dosage form, wherein the first region is free, or substantially free of the nucleotide reverse transcriptase inhibitor, and the second region is free or substantially free of the nucleoside reverse transcriptase inhibitor.

3. A pharmaceutical formulation according to claim 1 or 2, which is in the form of a multi-layered tablet, a first layer of said tablet comprising said nucleoside reverse transcriptase inhibitor and a second layer of said tablet comprising said nucleotide reverse transcriptase inhibitor wherein said first and/or second layer further comprises at least one pharmaceutically acceptable excipient.

4. A pharmaceutical formulation according to claim 3, wherein the multi-layered tablet is a bilayered tablet or a trilayered tablet.

5. A pharmaceutical formulation according to claim 4, wherein the multi-layered tablet is a trilayered tablet, and wherein a third layer of said tablet is intermediate between said first and second layers, and is optionally free or substantially free of the nucleoside reverse transcriptase inhibitor and the nucleotide reverse transcriptase inhibitor.

6. A pharmaceutical formulation according to claim 1 or 2, wherein the coated tablet is formed of a core comprising one of said first or second regions, and a coating layer comprising the other of said first or second regions.

7. A pharmaceutical formulation according to claim 6, wherein the coated tablet comprises an intermediate layer interposed between to said core and coating layer, and wherein the intermediate layer of said tablet is optionally free or substantially free of the nucleoside reverse transcriptase inhibitor and the nucleotide reverse transcriptase inhibitor.

8. A pharmaceutical formulation according to any preceding claim, wherein the nucleoside reverse transcriptase inhibitor is lamivudine, abacavir, emtricitabine, zidovudine or stavudine.

9. A pharmaceutical formulation according to any preceding claim, wherein the nucleotide reverse transcriptase inhibitor is tenofovir DF or adefovir.

10. A pharmaceutical formulation according to any preceding claim, wherein the nucleoside reverse transcriptase inhibitor is lamivudine and the nucleotide reverse transcriptase inhibitor is tenofovir DF.

11. A pharmaceutical formulation according to any preceding claim comprising from 50mg to 600mg of said nucleoside reverse transcriptase inhibitor, per unit dosage form, preferably from 150mg to 450mg of said nucleoside reverse transcriptase inhibitor, per unit dosage form, more preferably approximately 300mg of said nucleoside reverse transcriptase inhibitor, per unit dosage form; and further comprising from 75mg to 600mg of said nucleotide reverse transcriptase inhibitor, per unit dosage form, preferably from 150mg to 450mg of said nucleotide reverse transcriptase inhibitor, per unit dosage form, and more preferably approximately 300mg of said nucleotide reverse transcriptase inhibitor.

12. A pharmaceutical product comprising a pharmaceutical formulation according to any preceding claim, and further comprising a non-nucleoside reverse transcriptase inhibitor.

13. A pharmaceutical product according to claim 12, wherein the non-nucleoside reverse transcriptase inhibitor is efavirenz, nevirapine or delavirdine, preferably efavirenz.

14. A pharmaceutical product according to any of claims 12 or 13, wherein the non-nucleoside reverse transcriptase inhibitor is formulated as a first dosage form, and the nucleoside reverse transcriptase inhibitor and the nucleotide reverse transcriptase inhibitor are formulated as a second, separate, unitary dosage form.

15. A pharmaceutical product according to claim 12, wherein the nucleoside reverse transcriptase inhibitor is :
Lamivudine, the nucleotide reverse transcriptase inhibitor is tenofovir DF, and the a non-nucleoside reverse transcriptase inhibitor is efavirenz.

## Patentansprüche

1. Pharmazeutische Formulierung in einer Einzeldosierungseinheitsform, wobei die Dosierungsform umfasst:
(a) einen Nukleosid-Reverse-Transkriptase-Inhibitor, und
(b) einen Nukleotid-Reverse-Transkriptase-Inhibitor,
wobei die Formulierung in Form einer mehrschichtigen Tablette oder einer Tablette mit einem umhüllten Kern vorliegt und wobei der Nukleosid-Reverse-Transkriptase-Inhibitor in einem Bereich der Dosierungsform bereitgestellt ist, der von dem Bereich des Nukleotid-Reverse-Transkriptase-Inhibitors verschieden ist.

2. Pharmazeutische Formulierung nach Anspruch 1, wobei der Nukleosid-Reverse-Transkriptase-Inhibitor in einem ersten Bereich der Dosierungsform bereitgestellt ist und der Nukleotid-Reverse-Transkriptase-Inhibitor in einem zweiten Bereich der Dosierungsform bereitgestellt ist, wobei der erste Bereich frei oder im Wesentlichen frei von dem Nukleotid-Reverse-Transkriptase-Inhibitor ist und der zweite Bereich frei oder im Wesentlichen frei von dem Nukleosid-Reverse-Transkriptase-Inhibitor ist.

3. Pharmazeutische Formulierung nach Anspruch 1 oder 2, welche in Form einer mehrschichtigen Tablette vorliegt, wobei eine erste Schicht der Tablette den Nukleosid-Reverse-Transkriptase-Inhibitor umfasst und eine zweite Schicht der Tablette den Nukleotid-Reverse-Transkriptase-Inhibitor umfasst, wobei die erste und/oder zweite Schicht außerdem wenigstens einen pharmazeutisch verträglichen Exzipienten umfasst.

4. Pharmazeutische Formulierung nach Anspruch 3, wobei die mehrschichtige Tablette eine zweischichtige Tablette oder eine dreischichtige Tablette ist.

5. Pharmazeutische Formulierung nach Anspruch 4, wobei die mehrschichtige Tablette eine dreischichtige Tablette ist und wobei eine dritte Schicht der Tablette zwischen der ersten und der zweiten Schicht liegt und gegebenenfalls frei oder im Wesentlichen frei von dem Nukleosid-Reverse-Transkriptase-Inhibitor und dem Nukleotid-Reverse-Transkriptase-Inhibitor ist.

6. Pharmazeutische Formulierung nach Anspruch 1 oder 2, wobei die umhüllte Tablette gebildet ist aus einem Kern, der einen von den ersten oder zweiten Bereichen umfasst, und einer Hüllschicht, die den anderen von den ersten oder zweiten Bereichen umfasst.

7. Pharmazeutische Formulierung nach Anspruch 6, wobei die umhüllte Tablette eine Zwischenschicht umfasst, die zwischen dem Kern und der Hüllschicht eingeschoben ist, und wobei die Zwischenschicht der Tablette gegebenenfalls frei oder im Wesentlichen frei von dem Nukleosid-Reverse-Transkriptase-Inhibitor und dem Nukleotid-Reverse-Transkriptase-Inhibitor ist.

8. Pharmazeutische Formulierung nach einem der vorangehenden Ansprüche, wobei der Nukleosid-Reverse-Transkriptase-Inhibitor Lamivudin, Abacavir, Emtricitabin, Zidovudin oder Stavudin ist.

9. Pharmazeutische Formulierung nach einem der vorangehenden Ansprüche, wobei der Nukleotid-Reverse-Transkriptase-Inhibitor Tenofovir DF oder Adefovir ist.

10. Pharmazeutische Formulierung nach einem der vorangehenden Ansprüche, wobei der Nukleosid-Reverse-Transkriptase-Inhibitor Lamivudin ist und der Nukleotid-Reverse-Transkriptase-Inhibitor Tenofovir DF ist.

11. Pharmazeutische Formulierung nach einem der vorangehenden Ansprüche, umfassend 50 mg bis 600 mg des Nukleosid-Reverse-Transkriptase-Inhibitors, pro Dosierungseinheitsform, vorzugsweise 150 mg bis 450 mg des Nukleosid-Reverse-Transkriptase-Inhibitors, pro Dosierungseinheitsform, mehr bevorzugt ungefähr 300 mg des Nukleosid-Reverse-Transkriptase-Inhibitors, pro Dosierungseinheitsform; und außerdem umfassend 75 mg bis 600 mg des Nukleotid-Reverse-Transkriptase-Inhibitors, pro Dosierungseinheitsform, vorzugsweise 150 mg bis 450 mg des Nukleotid-Reverse-Transkriptase-Inhibitors, pro Dosierungseinheitsform, und mehr bevorzugt ungefähr 300 mg des Nukleotid-Reverse-Transkriptase-Inhibitors.

12. Pharmazeutisches Produkt umfassend eine pharmazeutische Formulierung nach einem der vorangehenden Ansprüche und außerdem umfassend einen Nicht-Nukleosid-Reverse-Transkriptase-Inhibitor.

13. Pharmazeutisches Produkt nach Anspruch 12, wobei der Nicht-Nukleosid-Reverse-Transkriptase-Inhibitor Efavirenz, Nevirapin oder Delavirdin, vorzugsweise Efavirenz ist.

14. Pharmazeutisches Produkt nach einem der Ansprüche 12 oder 13, wobei der Nicht-Nukleosid-Reverse-Transkriptase-Inhibitor als eine erste Dosierungsform formuliert ist und der Nukleosid-Reverse-Transkriptase-Inhibitor und der Nukleotid-Reverse-Transkriptase-Inhibitor als eine zweite, getrennte, einheitliche Dosierungsform formuliert sind.

15. Pharmazeutisches Produkt nach Anspruch 12, wobei der Nukleosid-Reverse-Transkriptase-Inhibitor Lamivudin ist, der Nukleotid-Reverse-Transkriptase-Inhibitor Tenofovir DF ist, und der Nicht-Nukleosid-Reverse-Transkriptase-Inhibitor Efavirenz ist.

## Revendications

1. Formulation pharmaceutique sous forme de dosage unitaire, la forme de dosage comprenant :
(a) un inhibiteur nucléosidique de la transcriptase inverse et
(b) un inhibiteur nucléotidique de la transcriptase inverse ;
la formulation étant sous la forme d'un comprimé multi-couches ou d'un comprimé avec un noyau enrobé et l'inhibiteur nucléosidique de la transcriptase inverse étant fourni dans une région différente de la forme de dosage de l'inhibiteur nucléotidique de la transcriptase inverse.

2. Formulation pharmaceutique selon la revendication 1, dans laquelle l'inhibiteur nucléosidique de la transcriptase inverse est fourni dans une première région de la forme de dosage et l'inhibiteur nucléotidique de la transcriptase inverse est fourni dans une seconde région de la forme de dosage, la première région étant exempte, ou sensiblement exempte, de l'inhibiteur nucléotidique de la transcriptase inverse, et la seconde région étant exempte ou sensiblement exempte de l'inhibiteur nucléosidique de la transcriptase inverse.

3. Formulation pharmaceutique selon la revendication 1 ou 2, qui est sous la forme d'un comprimé multi-couches, une première couche dudit comprimé comprenant ledit inhibiteur nucléosidique de la transcriptase inverse et une seconde couche dudit comprimé comprenant ledit inhibiteur nucléotidique de la transcriptase inverse, lesdites première et/ou seconde couches comprenant en outre au moins un excipient pharmaceutiquement acceptable.

4. Formulation pharmaceutique selon la revendication 3, dans laquelle le comprimé multi-couches est un comprimé à deux ou trois couches.

5. Formulation pharmaceutique selon la revendication 4, dans laquelle le comprimé multi-couches est un comprimé à trois couches et dans laquelle une troisième couche dudit comprimé est intermédiaire entre lesdites première et seconde couches, et est facultativement exempte ou sensiblement exempte de l'inhibiteur nucléosidique de la transcriptase inverse et de l'inhibiteur nucléotidique de la transcriptase inverse.

6. Formulation pharmaceutique selon la revendication 1 ou 2, dans laquelle le comprimé enrobé est formé d'un noyau comprenant l'une desdites première ou seconde régions, et d'une couche d'enrobage comprenant l'autre desdites première ou seconde régions.

7. Formulation pharmaceutique selon la revendication 6, dans laquelle le comprimé enrobé comprend une couche intermédiaire entreposée entre ledit noyau et la couche d'enrobage, et dans laquelle la couche intermédiaire dudit comprimé est facultativement exempte ou sensiblement exempte de l'inhibiteur nucléosidique de la transcriptase inverse et de l'inhibiteur nucléotidique de la transcriptase inverse.

8. Formulation pharmaceutique selon l'une quelconque des revendications précédentes, dans laquelle l'inhibiteur nucléosidique de la transcriptase inverse est la lamivudine, l'abacavir, l'emtricitabine, la zidovudine ou la stavudine.

9. Formulation pharmaceutique selon l'une quelconque des revendications précédentes, dans laquelle l'inhibiteur nucléotidique de la transcriptase inverse est le ténofovir DF ou l'adéfovir.

10. Formulation pharmaceutique selon l'une quelconque des revendications précédentes, dans laquelle l'inhibiteur nucléosidique de la transcriptase inverse est la lamivudine et l'inhibiteur nucléotidique de la transcriptase inverse est le ténofovir DF.

11. Formulation pharmaceutique selon l'une quelconque des revendications précédentes comprenant de 50 mg à 600 mg dudit inhibiteur nucléosidique de la transcriptase inverse, par forme de dosage unitaire, de préférence de 150 mg à 450 mg dudit inhibiteur nucléosidique de la transcriptase inverse, par forme de dosage unitaire, de manière plus préférable environ 300 mg dudit inhibiteur nucléosidique de la transcriptase inverse, par forme de dosage unitaire ; et comprenant en outre de 75 mg à 600 mg dudit inhibiteur nucléotidique de la transcriptase inverse par forme de dosage unitaire, de préférence de 150 mg à 450 mg dudit inhibiteur nucléotidique de la transcriptase inverse, par forme de dosage unitaire et de manière plus préférable environ 300 mg dudit inhibiteur nucléotidique de la transcriptase inverse.

12. Produit pharmaceutique comprenant une formulation pharmaceutique selon l'une quelconque des revendications précédentes et comprenant en outre un inhibiteur non nucléosidique de la transcriptase inverse.

13. Produit pharmaceutique selon la revendication 12, dans lequel l'inhibiteur non nucléosidique de la transcriptase inverse est l'éfavirenz, la névirapine ou la delavirdine, de préférence l'éfavirenz.

14. Produit pharmaceutique selon la revendication 12 ou 13, dans lequel l'inhibiteur non nucléosidique de la transcriptase inverse est formulé dans une première forme de dosage, et l'inhibiteur nucléosidique de la transcriptase inverse et l'inhibiteur nucléotidique de la transcriptase inverse sont formulés dans une seconde forme de dosage unitaire distincte.

15. Produit pharmaceutique selon la revendication 12, dans lequel l'inhibiteur nucléosidique de la transcriptase inverse est la lamivudine, l'inhibiteur nucléotidique de la transcriptase inverse est le ténofovir DF, et l'inhibiteur non nucléosidique de la transcriptase inverse est l'éfavirenz.
